# EUROPÄISCHE PATENTSCHRIFT

(11) **EP 3 914 195 B1**
(45) Veröffentlichungstag und Bekanntmachung des Hinweises auf die Patenterteilung: **29.07.2026**
(21) Anmeldenummer: 20702264.1
(22) Anmeldetag: 24.01.2020
(51) Int. Cl.: A61F 2/66, A61F 2/68, A61F 2/50

(54) **PROTHESENFUSSEINSATZ**
PROSTHETIC FOOT COMPONENT
INSERT PROTHÉTIQUE DE PIED

(30) Priorität: 25.01.2019 DE 102019101843
(43) Veröffentlichungstag der Anmeldung: 01.12.2021
(73) Patentinhaber: Ottobock SE & Co. KGaA, 37115 Duderstadt (DE)
(72) Erfinder: PUSCH, Martin, 37115 Duderstadt (DE)
(74) Vertreter: Gramm, Lins & Partner Patent- und Rechtsanwälte PartGmbB
(86) Internationale Anmeldenummer: PCT/EP2020/051780
(87) Internationale Veröffentlichungsnummer: WO 2020/152339

(56) Entgegenhaltungen:
- US-A- 5 116 384
- US-A1- 2005 060 045
- US-A1- 2006 041 321
- US-A1- 2015 190 247

## Beschreibung

Die Erfindung betrifft einen Prothesenfußeinsatz mit einer proximalen Befestigungseinrichtung zum Festlegen des Prothesenfußeinsatzes an einer Proximalkomponente oder einem Patienten, einem distal zu der Befestigungseinrichtung angeordneten und mit der Befestigungseinrichtung verbundenen Halter und einer Hauptfeder, die sich in einen Vorderfußbereich erstreckt und mit dem Halter gekoppelt ist.

Prothesenfußeinsätze sind Teil einer prothetischen Versorgung, beispielsweise bei Unterschenkelamputierten. Prothesenfußeinsätze können zur Erzielung einer möglichst natürlichen Optik sowie zur Bereitstellung einer weiteren Funktionalität mit einem Überzug oder einer Prothesenkosmetik versehen sein, die aus einem Kunststoff ausgebildet sein kann. Die Prothesenfußeinsätze können an einem Knöchelgelenk oder gelenklos an einem Unterschenkelrohr oder einem Unterschenkelschaft befestigt sein. Die Befestigungseinrichtung ist in der Regel als sogenannter Pyramidenadapter ausgebildet, über den eine Vielzahl von Einstellungen und Ausrichtungen des Prothesenfußeinsatzes relativ zu der Proximalkomponente, also dem Unterschenkelrohr, dem Prothesenschaft oder dem Knöchelgelenk eingestellt und fixiert werden können. Die Befestigungseinrichtung ist an einem Halter befestigt, an dem wiederum eine sich in Vorfußrichtung erstreckende Feder, beispielsweise eine Vorfußfeder oder Dachfeder angeordnet sein kann. Um einen Stoß bei einem Fersenauftritt abzudämpfen, ist ein elastisches Fersenelement vorgesehen, das an dem Halter befestigt ist, gegebenenfalls unter Einschaltung von Zwischenstücken. Beispiele für einen Protheseneinsatz sind in der EP 2 420 212 A1, der EP 1 976 463 A1, der US 2005/0038525 A1 oder der EP 2 688 522 B1 beschrieben.

Die US 2015/190247 A1 betrifft einen Prothesenfußeinsatz mit einer Sohlenstruktur, die in einem Zehenbereich von unten gesehen eine konvexe Wirkung aufweist, die im Mittelfußbereich in eine konkave Wölbung übergeht und sich bis zu einem Fersenbereich erstreckt. An einer Stützstruktur sind proximale Anschlussmittel angeordnet. Zwischen der Stützstruktur und der Sohlenstruktur ist ein elastisches Verbindungselement angeordnet, das einen horizontalen Schlitz aufweist, in den die Sohlenstruktur eingeführt ist. Zwischen der Sohlenstruktur und der Stützstruktur kann eine Steuerfeder angeordnet sein, die mit ihrem fersengerichteten Arm in das elastische Verbindungselement hineinragt.

In der US 5 116 384 A einem Prothesenfuß mit einer Sohlenfeder und einer Vorfußfeder, zwischen denen ein Pylon im hinteren Bereich angeordnet ist. Der Pylon ist in einem Kugelgelenk an dem Sohlenteil gelagert. In dem Mittelfußbereich und dem Vorfußbereich sind elastischer Elemente zwischen der Vorfußfeder und der Sohlenfeder angeordnet.

In der US 2006/041321 A1 ist ein Prothesenfuß mit einem einstellbaren Knöchel beschrieben. Hierzu ist ein oberes Element auf einem unteren Element, das mit einem Fußteil verbunden ist, verschieblich gelagert. Das obere Element ist mit einem gekrümmten Schlitz gleitend auf einer korrespondierenden gekrümmten Schiene gelagert. Dadurch kann der Winkel zwischen einem Unterschenkelteil und dem Fußteil eingestellt werden.

In der US 2005/060045 A1 ist ein Prothesenfuß beschrieben, bei dem ein oberes Element als Halter mit einem Fußteil kippbar verbunden ist. Zwischen dem Fußteil und dem oberen Element sind Dämpfer angeordnet, die eine Verlagerungsbewegung dämpfen.

Problematisch bei Prothesenfußeinsätzen aus dem Stand der Technik ist der gegebenenfalls benötigte Bauraum, ein unbefriedigendes Einsinkverhalten, ein ungleichmäßiges Abrollverhalten und Schwierigkeiten beim Ausgleichen von Unebenheiten. Darüber hinaus sind teilweise komplexe Formgebungen notwendig, die die Herstellkosten erhöhen und Schwierigkeiten bei der optimalen Materialausnutzung bereiten.

Aufgabe der vorliegenden Erfindung ist es daher, einen Prothesenfußeinsatz bereitzustellen, der ein optimiertes Verhalten beim Stehen als auch beim Gehen ermöglicht, insbesondere beim Gehen eine ausreichende Stabilität bereitstellt, ohne dass dies zu Lasten des Komforts beim Gehen geht.

Erfindungsgemäß wird diese Aufgabe durch einen Prothesenfußeinsatz mit den Merkmalen des Hauptanspruches gelöst. Vorteilhafte Ausgestaltung und Weiterbildungen der Erfindung sind den Unteransprüchen, der Beschreibung sowie den Figuren offenbart.

Der Prothesenfußeinsatz mit einer proximalen Befestigungseinrichtung zum Festlegen des Prothesenfußeinsatzes an einer Proximalkomponente einer Prothese oder einem Patienten selbst, einem distal zu der Befestigungseinrichtung angeordneten und mit der Befestigungseinrichtung verbundenen Halter und einer Hauptfeder, die sich in einen Vorderfußbereich erstreckt und mit dem Halter gekoppelt ist, sieht vor, dass der Halter in der Sagittalebene kippbar an der Hauptfeder gelagert ist, wobei ein posteriores Begrenzungselement zwischen der Hauptfeder und dem Halter angeordnet ist, das eine Verlagerung des Halters von der Hauptfeder weg begrenzt, wobei das Begrenzungselement mit einer Vorspannung zwischen 5% und 60% des Körpergewichtes des Nutzers vorgespannt ist. Der Prothesenfußeinsatz ist entweder als ein separates Bauteil ausgebildet, das an einer distalen Prothesenkomponente, beispielsweise einem Unterschenkelrohr oder einem Unterschenkelschaft festlegbar ist, oder als ein einstückig mit einer entsprechenden Befestigungseinrichtung zur Festlegung an dem Patienten oder dem Nutzer des Prothesenfußeinsatzes hergestellten Prothesenfußeinsatz ausgebildet, beispielsweise mit einer Einrichtung zum osseointegrierten Festlegen an einem Patienten oder aber als integraler Bestandteil eines Unterschenkelschaftes.

Der Prothesenfußeinsatz kann insbesondere als Basis für sonstige Aufbauten wie ein mechatronisches Gelenk, ein ML-Adapter, ein Adapter zum Einstellen der Absatzhöhen, eine hydraulische Gelenkeinheit oder dergleichen dienen.

Ein posteriores Begrenzungselement verhindert, dass der Halter über eine einmal eingestellte Begrenzung hinaus von der Hauptfeder weg verlagert wird. Das posteriore Begrenzungselement definiert den maximalen Abstand eines posterioren Endes des Halters zu der Hauptfeder, insbesondere zu einem posterioren Ende der Hauptfeder, die umgekehrte Bewegung bleibt jedoch weiter möglich. Dadurch wird einerseits eine Einfederung des Prothesenfußeinsatzes bei einer Fersenbelastung oder bei einem Heel Strike nicht oder nur vernachlässigbar beeinträchtigt und andererseits bei einer Abrollbewegung oder beim Nachvornebeugen während des Stehens eine ausreichende Stabilität bei einer Vorfußbelastung bereit gestellt, so dass neben einem grundsätzlich möglichem elastischen Nachgeben beim Stehen eine Verkippung in der Sagittalebene in anteriorer Richtung um eine Kippachse begrenzt wird.

Die Hauptfeder ist als eine zusammengesetzte Feder mit zumindest einer Distalfeder und/oder zumindest einer Proximalfeder ausgebildet. Bei beispielsweise zwei Distalfedern bildet die proximale Distalfeder eine Medialfeder und die distale Distalfeder eine Basisfeder oder eine Bodenfeder aus, die sich bevorzugt bis in den Fersenbereich des Prothesenfußeinsatzes erstreckt. Die Proximalfeder ist dem Halter oder einem Führungselement für den Halter zugeordnet. Alle Federn sind insbesondere als Blattfedern ausgebildet. Die Blattfedern können einen im Wesentlichen rechteckigen Querschnitt aufweisen und in Längserstreckung eine gleichmäßige Dicke oder auch ein sich verändernde, insbesondere in anteriorer Richtung verjüngende Dicke aufweisen. Die Federn können im Vorderfußbereich geschlitzt ausgebildet sein, um eine medial-lateral Verkippung des Prothesenfußes zu ermöglichen oder um eine Öffnung oder Einkerbung auszubilden, beispielsweise um einen Zehenriemen einer Sandale oder ähnliches aufnehmen zu können.

Die Distalfeder und die Proximalfeder der Hauptfeder sind bevorzugt unter Ausbildung eines Freiraumes beabstandet zueinander aneinander festgelegt, um ein Einfedern des Prothesenfußes bei einer Belastung zu ermöglichen und die einzelnen Federeigenschaften der separaten Federn auszunutzen. Die Federn sind insbesondere aus einem Kompositwerkstoff, insbesondere einem faserverstärkten Kunststoff ausgebildet. Die Federn können aus in einer Matrix eingebetteten Glasfasern, Karbonfasern, Aramidfasern, Kevlarfasern, Dyneemafasern oder anderen insbesondere hochfesten Fasern oder aus Kombinationen davon hergestellt werden.

Gemäß der Erfindung sind die Distalfeder und die Proximalfeder bi-konvex geformt zueinander ausgerichtet, um einen vergrößerten Freiraum im Mittelbereich zwischen den beiden Federn auszubilden. Der Freiraum ist angenähert elliptisch ausgebildet und ermöglicht ein Einfedern über einen vergleichsweise langen Federweg im Mittelfußbereich oder Vorderfußbereich. Die Proximalfedern können ebenfalls bikonvex zueinander geformt ausgebildet und ausgerichtet sein. Bei einer bikonvexen Formgebung zwischen der Distalfeder und der Medialfeder kann sich im posterioren Bereich zwischen der Distalfeder oder Basisfeder und der Medialfeder ein sich in posteriorer Richtung vergrößernder Zwischenraum ausgebildet sein, um ein tiefes Einfedern des Halters in Richtung auf die Basisfeder oder Bodenfeder zu ermöglichen.

Eine Weiterbildung der Erfindung sieht vor, dass ein Führungselement an der Hauptfeder in einem anterioren Bereich oder einem posterioren Bereich befestigt ist und sich in die jeweils entgegengesetzte Richtung erstreckt und der Halter über dieses Führungselement schwenkbar, bevorzugt um einer Schwenkachse orthogonal zu der Sagittalebene, an der Hauptfeder gelagert ist. Durch die Lagerung des Halters an der Hauptfeder über ein Führungselement lassen sich unterschiedliche Lagerungs- und Bewegungsvarianten einstellen und verändern, so dass eine individuelle Anpassung oder eine Vergrößerung der Anzahl an Varianten erreichen lässt. Der Halter ist dann nicht unmittelbar mit einer Hauptfeder verbunden und daran festgelegt, beispielsweise durch eine direkte Verschraubung einer Blattfeder an der Unterseite einer Halterbasis, sondern schwenkbar um eine Achse quer zur Gehrichtung, z.B. über das Führungselement, ein Scharnier oder ein anderes Zwischenelement, das eine Relativbewegung zwischen der Hauptfeder und dem Halter zulässt. Das Führungselement kann in einem anterioren oder posterioren Bereich der Hauptfeder befestigt sein, wobei der posteriore Bereich der Befestigungseinrichtung hinter der Befestigungseinrichtung oder einem Pyramidenadapter liegt, insbesondere posterior einer Krafteinleitungsstelle beim Stehen. Der anteriore Bereich liegt anterior der Befestigungseinrichtung, beispielsweise eines Pyramidenadapters oder der Position des resultierenden Bodenreaktionskraftvektors beim Stehen. Von dem jeweiligen Befestigungsbereich des Führungselementes erstreckt sich dieses in die jeweils entgegengesetzte Richtung, also bei einer Befestigung im posterioren Bereich in anteriore Richtung und bei eine Befestigung im anterioren Bereich in posteriore Richtung. Um das Führungselement wird der Halter kippbar in der Sagittalebene an der Hauptfeder gelagert, so dass eine Verschwenkbewegung ebenso wie eine vertikale Bewegung des Halters relativ zu der Hauptfeder durch das Führungselement ermöglicht wird.

Eine Weiterbildung sieht vor, dass ein anteriores Begrenzungselement zwischen dem Führungselement und dem Halter oder Hauptfeder angeordnet ist, das bei einer Fersenbelastung eine Verlagerung des anterioren Endes des Halters oder eine Verlagerung der Hauptfeder von dem Führungselement weg begrenzt. Wie bei dem posterioren Begrenzungselement wird durch das anteriore Begrenzungselement eine Relativverlagerung des Halters zu der Hauptfeder während einer bestimmten Belastungsphase verhindert, um das Einfederverhalten und Ausfederverhalten des Prothesenfußeinsatzes zu beeinflussen. Durch die Einstellung der Länge des anterioren und/oder posterioren Begrenzungselementes kann eine Federverspannung verändert werden und eine Anpassung des Energietransportverhaltens des Fußes während der verschiedenen Gangphasen erfolgen. Ebenso kann eine Anpassung an die unterschiedlichen Nutzungsgewohnheiten, Einsatzzwecke bei dem jeweiligen Nutzer oder an unterschiedliche Nutzer, an unterschiedliche oder sich verändernde körperliche Eigenschaften der jeweiligen Nutzer und dergleichen durch Veränderung der Vorspannung und/oder der Länge zur Begrenzung der Verlagerung eines anterioren und/oder posterioren Endes des Halters erfolgen.

Das jeweilige Begrenzungselement kann zugstarr und flexibel ausgebildet sein, um eine präzise Einstellung der möglichen Verlagerung und der Abstände von Halter zur Feder oder von der Feder zu dem Begrenzungselement oder Führungselement zu gewährleisten. Das Begrenzungselement ist beispielsweise als Gurt, Kabel oder Schnur oder auch als Hülsenführung mit einem Anschlagelement zur jeweiligen Begrenzung des Maximalabstandes ausgebildet.

Die Hauptfeder und das Führungselement sind bevorzugt als Blattfedern ausgebildet, insbesondere als gerade Blattfedern, was den Vorteil hat, dass die Herstellung der Federkomponenten besonders einfach ist. Insbesondere wenn die Hauptfeder oder die einzelnen Federkomponenten der Hauptfeder aus einem faserverstärkten Kunststoff hergestellt sind, kann die Hauptfeder insgesamt vergleichsweise steif ausgebildet werden, so dass sich die Haltbarkeit erhöht. Eine steife Federauslegung der einzelnen Federkomponenten ermöglicht eine hohe Haltbarkeit der Federkomponenten, die steife Federauslegung führt jedoch nicht zu einem besonders steifen Prothesenfußeinsatz beim Fersenauftritt, da dies über den vergleichsweise großen Federweg und eine lange Kraftdurchleitung kompensiert werden kann. Das Führungselement kann ebenfalls als Blattfeder ausgebildet sein, insbesondere als eine metallische Blattfeder in Gestalt einer Federzunge. Alternativ kann der Halter über ein Scharnier oder zumindest ein Abstandselement auf der Hauptfeder gelagert sein, so dass das Führungselement als Federzunge, Scharnier oder Abstandselement ausgebildet ist.

Zur weiteren Einstellung des Gehverhaltens sowie zu einer verbesserten Anpassbarkeit des Abrollverhaltens sowie eines Schutzes der Distalfeder oder der distalen Abschnitte der Hauptfeder kann ein Vorderfußpolster und/oder ein Fersenpolster an der Hauptfeder befestigt sein, beispielsweise verklebt, verschraubt oder aufgesteckt oder über das jeweilige Begrenzungselement, insbesondere bei einem Fersenpolster gehalten werden. Die Polster sind bevorzugt an der Basisfeder oder Bodenfeder angeordnet.

Eine Weiterbildung der Erfindung sieht vor, dass die Kräfte von dem Halter auf die Hauptfeder über einen anterioren Krafteinleitungsbereich und einen posterioren Krafteinleitungsbereich eingeleitet werden, wobei sich die Krafteinleitungsbereiche jeweils anterior oder posterior der Befestigungseinrichtung erstrecken. Dadurch ist es möglich, dass sich der Halter an zwei in Längserstreckung der Hauptfeder beabstandeten Punkten oder Bereichen auf der Hauptfeder abstützt, ggf. über Zwischenelemente, Zwischenplatten oder auch eine Dämpfereinrichtung, gegebenenfalls auch über eine Zwischenfeder. Die Abstützung in diesen Krafteinleitungsbereichen ermöglicht eine Verringerung der punktuellen Belastung sowie eine gesteuerte Krafteinleitung bei unterschiedlichen Belastungsszenarien. Bevorzugt liegen die Krafteinleitungsbereiche zwischen zwei Endauflagern, auf denen die Hauptfeder sich im distalen Bereich abstützt, beispielsweise zwischen dem Fersenpolster und dem Vorderfußpolster. Dadurch findet zumindest eine Vier-Punkt-Biegung statt, wenn sich der Patient in der Standphase befindet und der Fuß aufgesetzt ist, so dass das maximale Biegemoment der Hauptfeder wesentlich reduziert wird, da die Krafteinleitung breiter verteilt ist.

Zumindest einer der Krafteinleitungsbereiche kann verlagerbar oder auswechselbar an dem Halter oder Hauptfeder gelagert sein, so dass durch eine Verstellung der Position des jeweiligen Krafteinleitungsbereiches eine Veränderung der Federeigenschaften sowie der Energietransporteigenschaften des Prothesenfußeinsatzes während der Benutzung erfolgen kann. Die Einstellung der jeweiligen Position des Krafteinleitungsbereiches erfolgt bevorzugt einmalig zur Anpassung des Verhaltens des Prothesenfußeinsatzes an die jeweiligen Nutzer und kann vor der jeweiligen Benutzung eingestellt und angepasst werden. Grundsätzlich ist es auch möglich die Position des Krafteinleitungsbereiches motorisch zu verändern. Der jeweilige Motor oder Antrieb kann dann über eine Steuereinrichtung und eine Sensoranordnung während des Gehens verstellt werden, um eine Anpassung an unterschiedliche Geschwindigkeiten, Belastungen oder Gehsituationen zu ermöglichen.

Zwischen dem Halter und der Hauptfeder kann zumindest eine Dämpfeinrichtung angeordnet sein, um nach einem Ablösen des Halters im anterioren Bereich oder im posterioren Bereich von der Hauptfeder den erneuten Kontakt zwischen Halter und Hauptfeder sanft auszugestalten oder um eine Verschwenkbewegung zu dämpfen. Damit wird während des Gehens das Auftreten störender Kraftspitzen und Impulse durch den Kontakt von Halter und Hauptfeder vermieden, was das Abrollverhalten für den Patienten ungleichmäßig und unangenehm machen kann. Ebenso kann das Verschwenkverhalten des Halters relativ zu der Hauptfeder eingestellt werden.

Der Halter kann mit einem einstellbaren Proximal-Distal-Abstand an der Hauptfeder gelagert sein, um eine Winkeleinstellung ebenso wie eine Veränderung des Eingriffsverhaltens und Eingriffszeitpunktes und damit eine Veränderung des Energieübergangs zu ermöglichen.

Eine Weiterbildung der Erfindung sieht vor, dass im unbelasteten Zustand des Prothesenfußeinsatzes die Hauptfeder gegenüber dem Führungselement durch das Begrenzungselement und/oder die Begrenzungselemente elastisch vorgespannt ist, so dass die einzelnen Komponenten des Prothesenfußeinsatzes im unbelasteten Zustand allein durch die Vorspannung der Hauptfeder relativ zu dem Führungselement zusammengehalten werden. Die Begrenzungselemente oder das Begrenzungselement wirken zwischen dem Halter und der Hauptfeder. Zwischen der Hauptfeder und dem Halter ist das Führungselement angeordnet und sofern nur vertikale Kräfte auftreten, also keine Verschiebungskräfte in horizontaler Richtung, bleibt der Prothesenfußeinsatz ohne weitere Sicherungseinrichtungen der Komponenten aneinander stabil. Weitere Sicherungseinrichtung oder Befestigungselemente dienen nur zur Sicherung gegenüber auftretenden Querkräften oder Scherkräften. Die Befestigungseinrichtung kann verschieblich und/oder gelenkig an dem Halter gelagert sein, um eine Anpassung an unterschiedliche Anforderungen oder Patienten vornehmen zu können.

Zwischen der Befestigungseinrichtung und dem Halter kann ein Dämpfer angeordnet sein, sofern die Befestigungseinrichtung als separates Element an dem Halter festgelegt ist. Der Dämpfer kann eine leichte Relativbewegung zwischen der Befestigungseinrichtung und dem Halter zulassen und Belastungsspitzen abbauen und eine gleichmäßige Abrollbewegung ohne Impulse ermöglichen.

Eine Weiterbildung der Erfindung sieht vor, dass zwischen der Proximalfeder und der Distalfeder und/oder zwischen den Distalfedern ein auswechselbares und/oder verschieblich gelagertes Kontaktelement angeordnet ist, über das der jeweilige Krafteinleitungspunkt und ein Kopplungszeitpunkt und Kopplungsort zwischen der Distalfeder und der Proximalfeder oder den Distalfedern festgelegt, eingestellt oder verstellt werden kann. Durch das auswechselbare und/oder verschieblich gelagerte Kontaktelement kann auf eine einfache Art und Weise eine Veränderung und Anpassung des Einfederverhaltens und Kraftübertragungsverhaltens des Prothesenfußeinsatzes erfolgen.

Der Halter kann in der Sagittalebene auf einer ortsveränderlichen Fläche kippbar an der Hauptfeder gelagert sein, so dass der Halter auf der Hauptfeder während der Belastung abrollen und eine Verlagerung relativ zu der Hauptfeder zulassen kann. Der Halter hat dadurch keinen festen Drehpunkt gegenüber der Hauptfeder, vielmehr verschiebt sich der Drehpunkt des Halters während des Abrollvorgangs.

Der Prothesenfußeinsatz ist insbesondere auch dafür geeignet und vorgesehen, um an unterschiedliche Absatzhöhen oder gewünschte Neigungswinkel angepasst zu werden, so dass der Nutzer einfach eine individuelle Anpassung vornehmen kann. Dies kann insbesondere durch eine Veränderung der Länge eines oder mehrerer Begrenzungselemente erfolgen. Das jeweilige Begrenzungselement kann ausgetauscht oder verlängert oder verkürzt und anschließend in der gewünschten Länge fixiert, z.B. geklemmt werden. Der Halter kann in der gewünschten Winkelstellung zu dem Boden oder der Hauptfeder eingestellt und fixiert werden, z.B. durch Austauschen von Polstern, Dämpfereinrichtungen, Zwischenstücken, Einleger oder Distanzelementen zwischen dem Halter und der Hauptfeder, um dadurch eine optimierte Ausrichtung des Halters und der Befestigungseinrichtung zu erreichen. Aufgrund der schwenkbaren Lagerung entweder vor oder hinter der Vertikalen durch die Befestigungseinrichtung kann durch Ausrichten und Feststellen in der gewünschten Position die Anpassung an die jeweilige Absatzhöhe erreicht werden. Die Position der Lagerstellen oder Achsen kann verstellbar ausgebildet sein, um eine Ausrichtung und Absatzhöhenanpassung zu erreichen. Sind Hydraulik- oder Pneumatikdämpfer oder Stellzylinder vorhanden, kann durch Öffnen und Schließen von Ventilen die gewünschte Stellung und Orientierung des Halters eingestellt werden. Der Halter ist mit einem einstellbaren proximal-distal-Abstand an der Hauptfeder gelagert, so dass bei einer gleichmäßigen Veränderung des Abstandes eine Anpassung an unterschiedliche Sohlendicken bei Schuhen stattfinden kann. Wird der Abstand vor und hinter der Befestigungseinrichtung unterschiedlich verändert, wird die Neigung des Halters wie gewünscht angepasst. Dazu können der vordere und hintere Abstand durch Einstellen von Stellzylindern oder Dämpfereinrichtungen oder durch Einleger oder dergleichen, wie oben beschrieben, individuell eingestellt werden.

Nachfolgend werden Ausführungsbeispiele der Erfindung anhand der beigefügten Figuren näher erläutern. Es zeigen:
- Figur 1 -: eine erste Ausführungsform des Prothesenfußeinsatzes in einer schematischen Schnittdarstellung;
- Figur 2-: eine Variante der Figur 1 mit einem Scharnier;
- Figur 3 -: eine Variante der Figur 1 mit einem gefalteten Führungsele-ment,
- Figur 4 -: eine Variante der Erfindung mit einem posterior überstehenden Halter;
- Figuren 5a - 5c -: eine Variante der Figur 1 mit Polstern in unterschiedlichen Belastungszuständen;
- Figur 6 -: eine Variante der Erfindung mit einem Polster zwischen dem Führungselement und der Hauptfeder;
- Figur 7 -: eine Variante der Figur 6;
- Figur 8 -: eine Variante mit einem Scharnier und zwei Federn;
- Figur 9 -: eine Variante der Figur 8;
- Figur 10 -: eine Variante mit einer Höheneinstellvorrichtung im Halter;
- Figur 11 -: eine Variante mit zwei im Wesentlichen parallel geführten Federn und einer entgegengesetzt gekrümmten Basisfeder;
- Figur 12 -: eine Variante mit einem verlagerbaren Kontakt zwischen Halter und Hauptfeder;
- Figur 13 -: eine Variante mit einer Umhüllung;
- Figur 14 -: eine Variante der Erfindung mit einem Verzögerungselement;
- Figur 15 -: ein Prothesenfußeinsatz nach Fig. 14 bei Belastung; sowie
- Figur 16 -: ein Prothesenfußeinsatz nach Fig. 14 in verkippter Stellung.

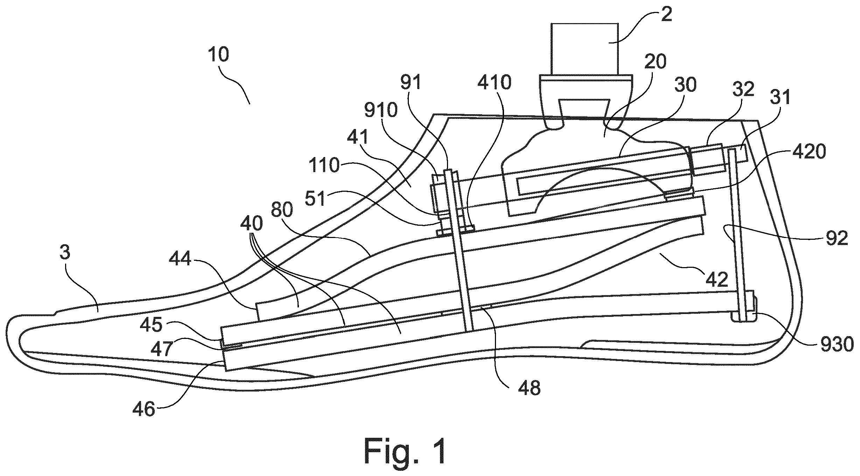

Figur 1 zeigt in einer schematischen Schnittdarstellung einen Prothesenfußeinsatz 10 mit einer proximalen Befestigungseinrichtung 20 in Gestalt eines Pyramidenadapters, der an einer Proximalkomponente 2 in Gestalt eines Unterrohrs reversibel festgelegt ist. Statt einer Befestigungseinrichtung 20 in Gestalt des Pyramidenadapters ist es möglich, dass die Befestigungseinrichtung 20 eine Aufnahme für einen Unterschenkelstumpf oder eine osseointegrierte Anbindung in einen Knochen aufweist. Die Befestigungseinrichtung 20 kann beispielsweise im Rahmen eines additiven Herstellverfahrens einstückig ausgebildet werden und gleichzeitig eine Stumpfaufnahme bilden. Ebenso ist es möglich, dass die Proximalkomponente 2 einstückig an der Befestigungseinrichtung 20 ausgebildet ist, beispielsweise ebenfalls über ein additives Herstellverfahren.

Distal zu der Befestigungseinrichtung 20 ist ein Halter 30 angeordnet, der einstückig mit der Befestigungseinrichtung 20 ausgebildet sein kann oder mit einer separat gefertigten Befestigungseinrichtung 20 verbunden ausgebildet sein kann. Der Halter 30 weist eine verstellbare Aufnahme 31 auf, die posterior über den Halter 30 hinausragt und zur Aufnahme eines posterioren Begrenzungselementes 92 dient. Die Länge des Halters 31 kann über eine Mutter 32, die gegenüber dem Halter 30 verspannt werden kann, eingestellt fixiert werden.

Der Halter 30 ist an einer Hauptfeder 40, die im dargestellten Ausführungsbeispiel aus drei Blattfedern 44, 45, 46 aufgebaut ist, in einem anterioren Bereich 41 und einem posterioren Bereich 42 befestigt. Im dargestellten Ausführungsbeispiel ist der Halter 30 über einen anterioren Krafteinleitungsbereich 410 und einen posterioren Krafteinleitungsbereich 420 auf der Proximalfeder 44 gelagert. Zwischen dem anterioren Krafteinleitungsbereich 410 und dem Halter 30 ist ein anteriores Dämpfungselement 50 angeordnet, das auf einer Aufnahme 910 für ein anteriores Begrenzungselement 91 aufliegt. Zwischen dem Krafteinleitungsbereich 410 und der Proximalfeder 44 unterhalb des Dämpfers 51, der beispielsweise als ein Elastomerdämpfer ausgebildet sein kann, wird eine Schwenkachse 110 ausgebildet, die sich im Wesentlichen senkrecht zu der Blattebene oder orthogonal zu der Sagittalebene im Wesentlichen horizontal erstreckt. Um diese Drehachse 110 kann der Halter 30 relativ zu der Hauptfeder 40, insbesondere zu der Proximalfeder 44 verkippt werden. Sowohl das anteriore Begrenzungselement 91 als auch das posteriore Begrenzungselement 92 sind unterhalb der Distalfeder 46 oder der Basisfeder 46 herumgeführt und weisen in dem dargestellten, nicht belasteten Zustand des Prothesenfußeinsatzes 10 eine Vorspannung auf, die durch die Verformung der Blattfedern 44, 45, 46 hervorgerufen wurde. Durch die Vorspannung werden alle Komponenten des Prothesenfußeinsatzes 10 aneinandergehalten. Im Bereich des anterioren Begrenzungselementes 91 ist zwischen der hinteren Blattfeder 45 oder Medialfeder 45 und der Distalfeder 46 ein Kontaktelement 48 angeordnet, dessen Position in anterior-posterior-Richtung verlagerbar ist. Das Kontaktelement 48 kann ausgewechselt werden, beispielsweise um den Ort der Krafteinleitung zwischen der Medialfeder 45 und der Distalfeder 46 zu bestimmen. Je nach Position in Richtung anterior oder posterior wird sich das Einfederverhalten aufgrund unterschiedlicher Krafteinleitungspunkte ändern. Auch zwischen dem anterioren Ende der Medialfeder 45 und dem anterioren Ende der Distalfeder 46 ist ein Kontaktelement 47 angeordnet, das verschieblich oder auswechselbar daran gelagert ist. Über die Materialauswahl, Größe und Position der Kontaktelemente 47, 48 können die Federeigenschaften des Prothesenfußeinsatzes 10 eingestellt werden.

Zwischen der Proximalfeder 44 und dem Halter 30 ist zudem ein Führungselement 80 angeordnet, das im posterioren Bereich 42, also an dem Bereich, der posterior der Krafteinleitung in die Befestigungseinrichtung 20 liegt, an dem Halter 30 festgelegt ist, beispielsweise verschraubt, verklebt, verschweißt, formschlüssig festgelegt oder geklemmt. Das Führungselement 80 erstreckt sich über den anterioren Krafteinleitungsbereich 410 hinaus bis zu einem vorderen Endbereich der Proximalfeder 44 und ist als Federblech ausgebildet, um eine Verkippung des Halters 30 relativ zu der Hauptfeder zu ermöglichen und ein Abrollen des Halters 30 auf der Oberseite der Proximalfeder 44 im Bereich des anterioren Krafteinleitungsbereiches 410 zu ermöglichen.

Die Begrenzungselemente 91, 92 können insbesondere als Gurte, Seile oder Kabel ausgebildet sein, sie können als Schlaufen ausgebildet und um die Oberseite bzw. Unterseite des Halters bzw. der Basisfeder 47 herum geführt sein. Ebenfalls besteht die Möglichkeit, dass die Begrenzungselemente 91, 92 als teleskopierbare Hülsen oder flexible und zugstarre Seile zwischen Aufnahmen an dem Halter 30 und der Basisfeder 47 gespannt gehalten sind. Der Prothesenfußeinsatz 10 ist einer Fußkosmetik 3 eingebettet und kann daran auswechselbar befestigt sein.

Die Begrenzungselemente 91, 92 können beide oder einzeln verstellbar, insbesondere verkürzbar oder verlängerbar ausgebildet sein. Alternativ kann durch Einlege- oder Distanzstücke eine Vorspannung zwischen dem oder den Begrenzungselementen 91, 92 und dem Halter 30 und/oder der Basisfeder 46 die Vorspannung variiert werden. Alternativ kann eine Veränderung der Vorspannung durch Auswechseln unterschiedlich langer Begrenzungselemente 91, 92 erfolgen. Um ein Abrutschen bei einer Entlastung oder Aufhebung der Vorspannung des Begrenzungselementes 91, 92 zu verhindern, kann das Begrenzungselement 91, 92 formschlüssig in der jeweiligen Aufnahme festgelegt sein. Beispielsweise ist an dem posterioren Ende der Basisfeder 46 eine solche Aufnahme 93 mit einer Durchführung oder einer Einrichtung zum Festlegen des Begrenzungselementes 92 vorgesehen. Die Aufnahme 93 kann gleichzeitig eine Polsterfunktion aufnehmen.

Wird eine über das Stehen hinausgehende Fersenlast aufgebracht, beispielsweise beim Heel Strike, federn die drei Federn 44, 45, 46 der Basisfeder 40 dergestalt ein, dass der Halter 30 über den posterioren Krafteinleitungsbereich 42 eine Axialkraft in Richtung auf den Boden ausübt und die Hauptfeder 44 im posterioren Bereich komprimiert, sodass das posteriore Begrenzungselement 92 entlastet wird, während das anteriore Begrenzungselement 91 weiterhin gespannt bleibt. Im weiteren Verlauf der Fußbelastung wird bei einer im Wesentlichen senkrechten Krafteinleitung über die proximale Komponente 2 in die Befestigungseinrichtung 20 eine gleichmäßige Kompression insbesondere der Proximalfeder 44 und der Medialfeder 45 vorgenommen, sodass beide Begrenzungselemente 91, 92 entspannt werden. Wird dann im Rahmen eines Gangfortschrittes die Proximalkomponente in anteriorer Richtung, also nach vorne verkippt, hebt sich das posteriore Ende des Halters 30 von der Proximalfeder 44 ab, sodass sich eine Ablösung oder ein Spalt zwischen dem posterioren Krafteinleitungsbereich 420 und der Proximalfeder 44 einstellt. Der Halter 30 ist dann über das Führungselement 80 in Medial-Lateral-Richtung sowie gegen ein Verdrehen gegenüber der Hauptfeder 40 gesichert. Das Abrollen kann vergleichsweise einfach ohne einen sehr hohen Widerstand aufgrund der verkippbaren oder schwenkbaren Lagerung des Halters 30 auf der Hauptfeder 40 erfolgen. Sobald der Verschwenkwinkel so groß wird, dass der Abstand zwischen dem posterioren Ende des Halters 30 und dem posterioren Ende der Basisfeder 46 so groß wird, dass das posteriore Begrenzungselement 92 gespannt wird, wird die Basisfeder 46 zusätzlich aktiviert und stellt eine zusätzliche Gegenkraft gegen eine weitere Verschwenkung nach vorne zur Verfügung. Dadurch ist es möglich, eine geschaltete Aktivierung der einzelnen Federn 44, 45, 46 während eines Gangzyklusses bereitzustellen, da der maximale Verschwenkweg des Halters 30 relativ zu der Proximalfeder 44 begrenzt wird. Die Begrenzung wird über das Begrenzungselement 92 wirksam.

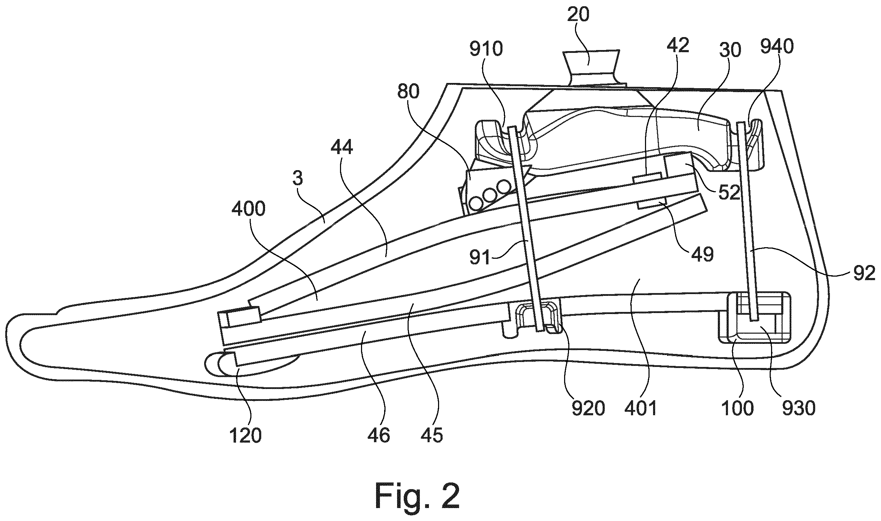

Figur 2 zeigt eine Variante des Prothesenfußeinsatzes 10 ebenfalls mit drei Federn 44, 45, 46, wobei die Proximalfeder 44 und die Medialfeder 45 bikone zueinander angeordnet sind, sodass sich ein elliptischer oder nahezu elliptischer Freiraum 400 ausbildet. Zwischen der Unterseite der Medialfeder 46 und der Oberseite der Distalfeder 45 ist ebenfalls im posterioren Bereich ein Freiraum 401 ausgebildet, um ein Einfedern beim Heel Strike zu ermöglichen.

Die Variante gemäß Figur 2 weist Aufnahmen 910 und 940 im anterioren und posterioren Bereich des Halters 30 auf, die als Nuten zur Aufnahme der jeweiligen Begrenzungselemente 91, 92 ausgebildet sind. An der Basisfeder 46 sind ebenfalls Aufnahmen 920, 930 ausgebildet, die als Schutz für die Basisfeder 46 dienen und gleichzeitig eine ungewollte Verlagerung des jeweiligen Begrenzungselementes 91, 92 verhindern. Die Aufnahme 930 am posterioren Ende der Basisfeder 46 weist ein Durchgangsloch auf, das eine Entfernung des Begrenzungselementes 92 auch bei einer Entlastung verhindert. Die Aufnahme 930 ist in einem Fersenpolster 100 ausgebildet, an dem anterioren Ende der Basisfeder 46 ist ein Vorfußpolster 120 angeordnet. Zwischen den Federn können jeweils auswechselbare oder verschiebliche Kontaktelemente angeordnet sein, die in dem dargestellten Ausführungsbeispiel nur zwischen dem posterioren Ende der Proximalfeder 44 und der Medialfeder 45 mit dem Bezugszeichen 49 angedeutet sind. Zwischen dem Halter 30 und der Proximalfeder 44 kann ein Dämpferelement 52 als Elastomerdämpfer angeordnet sein. Das anteriore Ende des Halters 30 ist über ein Scharnier mit der Feder 44 verbunden, sodass der Halter 30 relativ zu der Proximalfeder 44 verschwenken kann, sich jedoch nicht in vertikaler Richtung davon lösen kann. Der Lagerbock des Scharniers ist für die Aufnahme von Torsionskräften formschlüssig mit der Proximalfeder 44 verbunden, sodass sich eine Verdrehung um eine Vertikalachse nicht einstellen kann. Eine Verkippung um eine im Wesentlichen waagerechte Schwenkachse 110 senkrecht zu der Gehrichtung oder Längserstreckung des Prothesenfußeinsatzes ist jedoch möglich. Das Scharnier kann in einem posterioren Bereich 42 der Hauptfeder 40 befestigt sein, beispielsweise über ein Federblech oder ein Federelement oder aber im anterioren Bereich 41 über eine Klemmung oder eine andere, formschlüssige Festlegung.

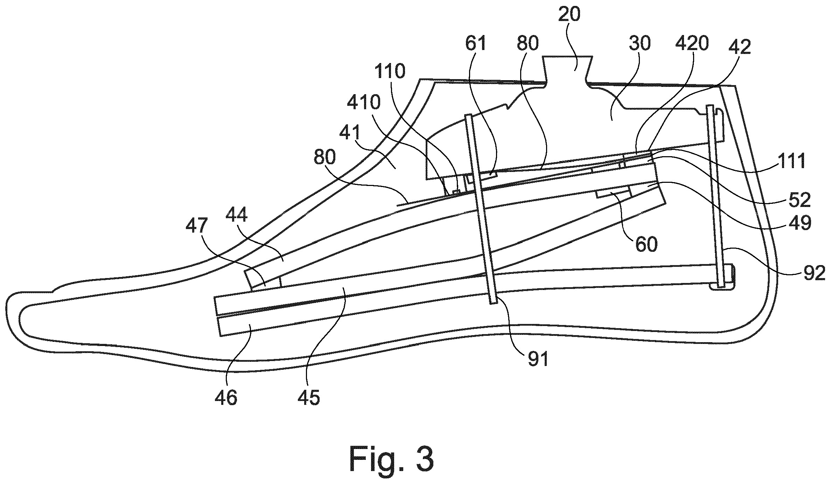

In der Figur 3 ist eine weitere Variante der Erfindung dargestellt, der Federaufbau entspricht im Wesentlichen dem der Figur 1 oder 2, wobei die Befestigung des Halters 30 über das Führungselement 80 an der Hauptfeder 40 abweicht. Während sich in der Figur 1 das Führungselement 80 von dem anterioren Endbereich der Proximalfeder 44 zum posterioren Endbereich des Halters 30 erstreckt, ist das dargestellte Ausführungsbeispiel mit einem gefalteten Führungselement 80 oder einem mehrteiligen Führungselement 80 versehen, das zunächst im anterioren Bereich 41 beispielsweise über einen Elastomerdämpfer 51 oder ein Befestigungselement an der Proximalfeder 44 festgelegt ist. Von dem anterioren Bereich 41 erstreckt sich das Führungselement 80 bis zum posterioren Bereich 42 der Proximalfeder 44 und von dort wieder nach vorn bis unter das anteriore Begrenzungselement 91 kurz vor den anterioren Dämpfer und wird dort über ein Befestigungselement 61 an der Unterseite des Halters 30 gehalten. Durch diese gefaltete Ausgestaltung des Führungselementes 80, das beispielsweise als Federblech ausgebildet sein kann, ist es möglich, eine Verkippung nach vorn um eine anteriore Schwenkachse 110 im Bereich des vorderen Krafteinleitungsbereich 410 durchzuführen und darüber hinaus eine Rückwärtsverkippung im posterioren Bereich 42 um eine hintere Schwenkachse 111 oberhalb des posterioren Dämpfers 52 im posterioren Krafteinleitungsbereich 420 zu ermöglichen. Diese hintere Schwenkachse 111 tritt beispielsweise bei einer Rückwärtsverlagerung bei vorhandener Axialbelastung in Funktion, bis das anteriore Begrenzungselement 91 durch eine entsprechende Verlagerung und Entspannung der Federn wirksam geschaltet wird. Statt einer einstückigen, gefalteten Ausführung des Führungselementes kann dieses auch zweiteilig ausgebildet und im hinteren, posterioren Bereich verbunden sein.

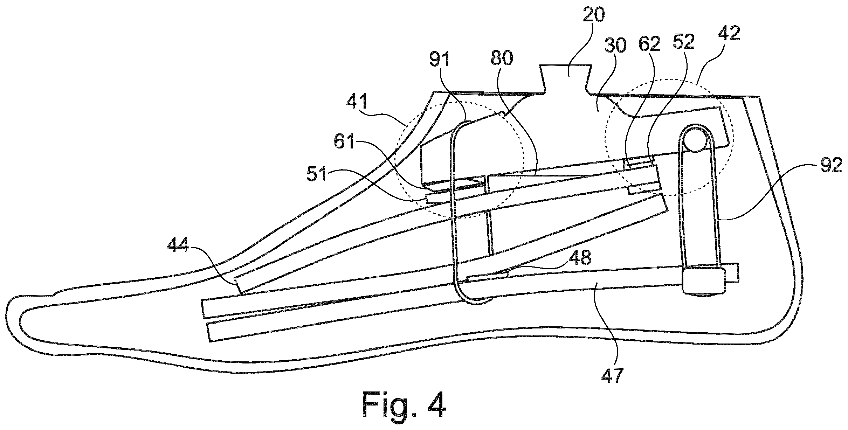

Eine weitere Variante der Erfindung ist in der Figur 4 dargestellt, bei der sich das Führungselement 80 von einem posterioren Bereich 42 auf der Hauptfeder 40, auf der es beispielsweise über ein Befestigungselement 62 in Gestalt einer Klammer oder Klemme gehalten wird, zum anterioren Bereich 41 des Halters 30 erstreckt und dort entweder klemmend gehalten oder über ein anteriores Befestigungselement 61 festgelegt wird. Zwischen den Befestigungselementen 61, 62 und der Proximalfeder 44 bzw. dem Halter 30 können Dämpfer 51, 52 angeordnet sein, um einen Kontakt zwischen Halter 30 und Feder 44 während des Gehens sanft zu gestalten. Die Begrenzungselemente 91, 92 sind als Schlaufen ausgebildet, die anteriore Schlaufe ist medial-lateral um den Prothesenfußeinsatz 10 herum geführt, das posteriore Begrenzungselement 92 ist medial und/oder lateral geführt, es können auch mehrere Begrenzungselemente 92 medial und/oder lateral an dem Halter 30 und der Basisfeder 47 angeordnet sein.

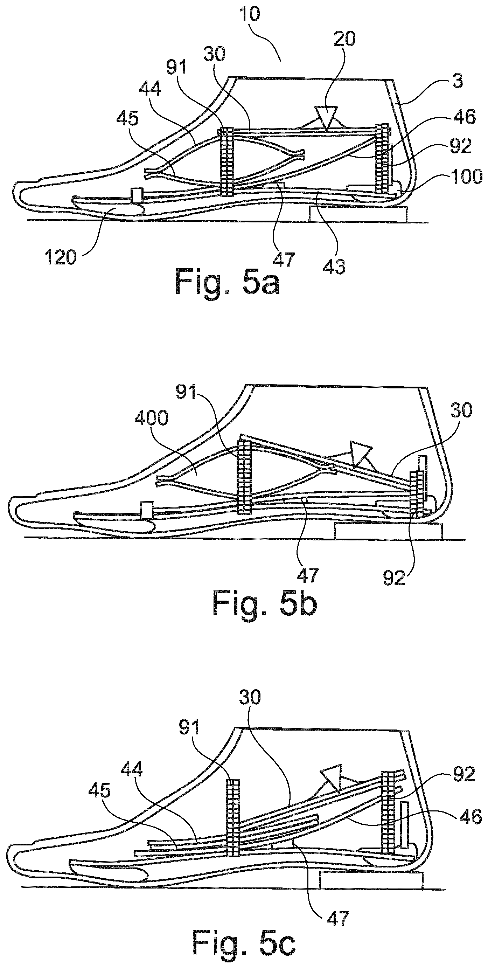

In den Figuren 5a bis 5c sind unterschiedliche Belastungsphasen eines Prothesenfußeinsatzes 10 in einer weiteren Ausführungsform dargestellt. Figur 5a zeigt den Prothesenfußeinsatz 10 innerhalb der Fußkosmetik 3 in einem unbelasteten Zustand oder beim entspannten Stehen mit einem im Wesentlichen waagerecht orientierten Halter 30, der sich an seinem vorderen Ende über eine Doppelfederanordnung mit einer Proximalfeder 44 und einer Medialfeder 45 auf einer Distalfeder 46 abstützt. Unterhalb der Distalfeder 46 ist eine Bodenfeder 43 angeordnet, an der ein Fersenpolster 100 und ein Vorderfußpolster 120 angeordnet sind. Die Distalfeder 46 und die Bodenfeder 43 sind an dem vorderen Ende der Distalfeder 46 aneinander befestigt, ungefähr im Bereich des Vorderfußpolsters 120. Die Distalfeder 46 kann sich bis zum Vorderfußende oder zum vorderen Ende der Bodenfeder 43 erstrecken. Zwischen der nach oben gekrümmten Distalfeder 46 und der Basisfeder 43 ist ein Kontaktelement 47 angeordnet, über das ein Krafteinleitungspunkt insbesondere bei einer Fersenbelastung eingestellt werden kann.

Durch die Vorspannung der beiden als Gurte ausgebildeten Begrenzungselemente 91, 92 kann die Steifigkeit des Prothesenfußeinsatzes 10 gegen ein Verkippen in anterior-posterior-Richtung eingestellt werden. Je größer die Vorspannung der Begrenzungselemente 91, 92 ist, desto steifer oder stabiler ist der Prothesenfußeinsatz.

In der Figur 5b ist die Anordnung und das Verhalten des Prothesenfußeinsatzes 10 bei einer starken Fersenbelastung schematisch gezeigt. Der hintere Teil des Halters 30 wird belastet und drückt auf das hintere Ende der Distalfeder 46, die in Richtung auf das posteriore Ende der Basisfeder 43 gedrückt wird. Da das vordere Ende der Distalfeder 46 an dem Vorderfußbereich der Basisfeder 43 festgelegt ist, wölbt sich die Distalfeder im Mittelfußbereich über das Kontaktelement 47, sodass sich eine Dreipunktbiegung einstellt. Darüber hinaus wird anterior zu dem Kontaktelement 47 eine senkrecht nach unten wirkende Kraft über die Doppelfeder 44, 45 aufgrund der Vorspannung durch das Begrenzungselement 91 ausgeübt. In der Figur 5b ist angedeutet, dass das Begrenzungselement 92 vollständig entspannt ist, posterior zu dem Halter 30 ist noch eine Führung sowohl für die Distalfeder 46 als auch für den Halter 30 angeordnet, die verhindert, dass das hintere Begrenzungselement 92 von dem Halter 30 abrutschen kann.

Bei einer Vorderfußbelastung, die beispielsweise während des Abrollens nach dem sogenannten roll over auftritt, wird der vordere, anteriore Bereich des Halters 30 belastet und stützt sich über die beiden Federn 44, 45 auf der Distalfeder 46 und über die Distalfeder 46 auf der Basisfeder 43 ab. Über das Kontaktelement 47 wird der Krafteinleitungspunkt definiert und durch Verschieben entlang der Längserstreckung der Federn verändert. Die Proximalfeder 44 und die Medialfeder 45 werden aufeinander zu bewegt, sodass der Freiraum 400 zwischen den beiden Federn verringert oder minimiert wird. Das hintere Ende des Halters 30 wird aufgrund der verschwenkbaren Lagerung auf der Proximalfeder 44 nach oben verlagert, bis es in Kontakt mit dem hinteren Begrenzungselement 92 tritt. In diesem Zustand hebt sich das hintere Ende des Halters 30 von der Distalfeder 46 ab.

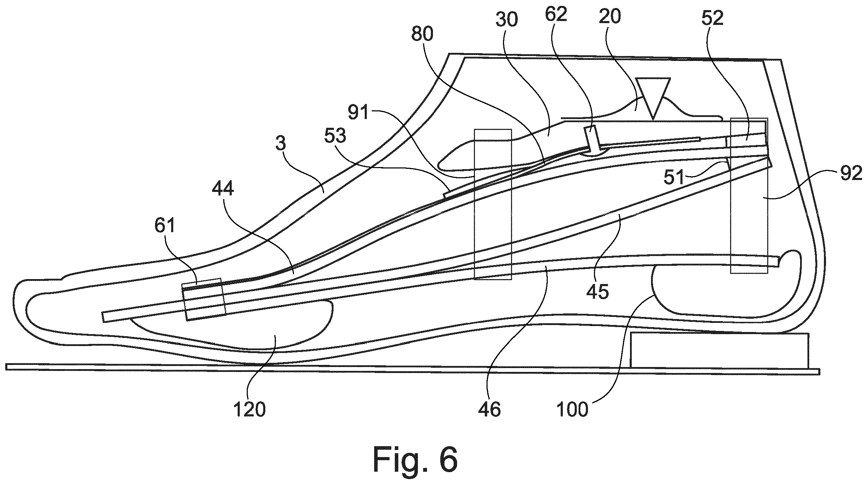

Figur 6 zeigt eine weitere Variante des Prothesenfußeinsatzes 10 mit einem Befestigungselement 80 in Gestalt einer Federzunge, die über eine Schraube 62 oder ein anderes Befestigungselement an dem Halter 30 an dessen Unterseite festgelegt ist. Der Halter 30 stützt sich über ein Polster 53 sowohl auf der Proximalfeder 44 als auch auf der Oberseite des Führungselementes 80 ab. Bei einer Vorfußbelastung verkippt der Halter im Bereich der Auflage des Polsters 53 um eine nicht definierte Schwenkachse herum, bis das hintere Begrenzungselement 92 eine Weiterverschwenkung relativ zu der Proximalfeder 44 verhindert. Das Führungselement 80 erstreckt sich bis zum vorderen Ende der Proximalfeder 44 und wird dort über ein Befestigungselement 61, beispielsweise eine Spange, eine Klammer oder einen Gurt gehalten. Die Distalfeder 46 endet ungefähr auf der gleichen Höhe wie die Proximalfeder 44. Die Medialfeder 45 erstreckt sich weiter in anteriorer Richtung über das Vorderfußpolster 120 hinaus. Im posterioren Bereich sind zwischen Halter 30 und Proximalfeder 44 sowie zwischen Proximalfeder 44 und Medialfeder 45 Dämpfer 51, 52 angeordnet, um bei einem Abheben der Federn bzw. Federn von dem Halter 51 bei einem Kontakt eine Dämpfung eines Impulses herbeizuführen. Die Dämpfer 51, 52 sind bevorzugt einseitig an dem Halter 30 oder einer der Federn 44, 45 befestigt, um eine Auseinanderbewegung der Komponenten voneinander zu ermöglichen. Bei hoch elastischen Materialien können die Dämpfer 51, 52 auch beidseitig mit den Komponenten verklebt werden.

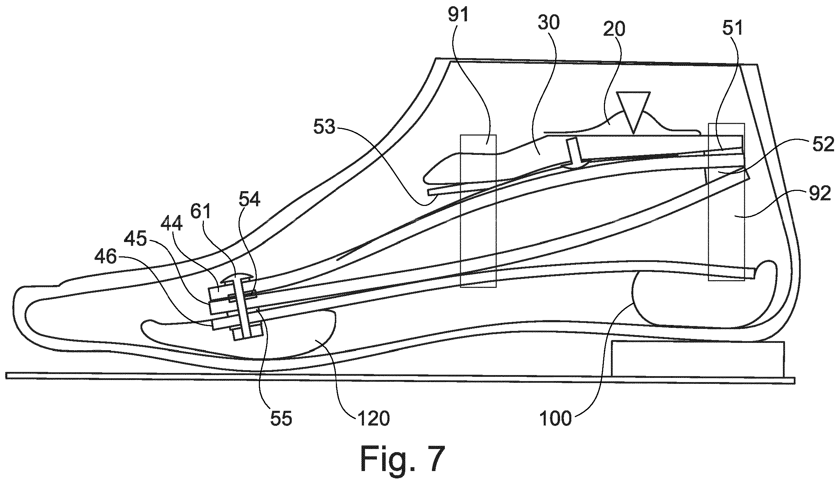

Figur 7 zeigt eine Variante der Figur 6, die einen grundsätzlich gleichartigen Aufbau aufweist, jedoch statt einer Spange oder einer Klammer als Befestigungselement 61 eine durch alle Federn 44, 45, 46 hindurchgehende Schraube aufweist, die eine Verschiebung der Federn 44, 45, 46 aufeinander entlang bei einer Belastung verhindert. Zwischen den Federn 44, 45, 46 sind jeweils Polster oder Dämpfer 54, 55 angeordnet, ebenso kann zwischen dem Kopf der Schraube 61 und der Proximalfeder 44 sowie der Mutter und der Distalfeder 46 ein Polsterelement angeordnet sein. Durch die Klemmung im Vorfußbereich wird den Federn 44, 45, 46 bei einer Verformung ein Moment überlagert, wodurch sich die freie Federlänge verkürzt. Die Verklemmung mit einer Schraube 61 verhindert eine Schubbewegung und macht insgesamt den Prothesenfußeinsatz 10 steifer als bei einer Lösung, die eine Verschiebung der Federn zueinander ermöglicht.

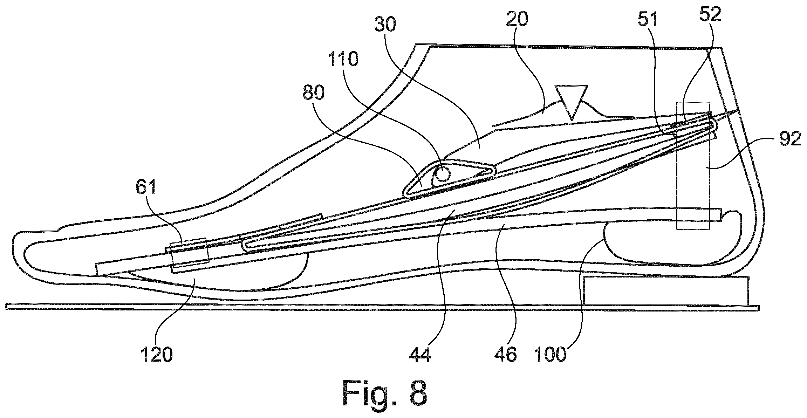

Figur 8 zeigt eine weitere Variante der Erfindung mit einem Führungselement 80, das als Scharnieraufnahme für den Halter 30 ausgebildet ist, und der um eine Schwenkachse 110 um sein vorderes Ende verschwenkt werden kann. Das hintere Ende des Halters 30 ist über das hintere Begrenzungselement 92 und seiner maximalen Verschwenkung hinsichtlich der Distalfeder 46 begrenzt. Das Führungselement 80 erstreckt sich bis in den Vorderfußbereich und ist über das Befestigungselement 61 zusammen mit der Distalfeder an der Basisfeder 46 angeordnet. Zwischen dem Halter 30 und dem Führungselement 80, das sich bis zum hinteren Ende des Halters 30 erstreckt, ist ein Dämpfer 51 angeordnet, ebenso wie zwischen dem Führungselement 80 und dem hinteren Ende der Proximalfeder 44 ein weiterer Dämpfer 52 angeordnet ist.

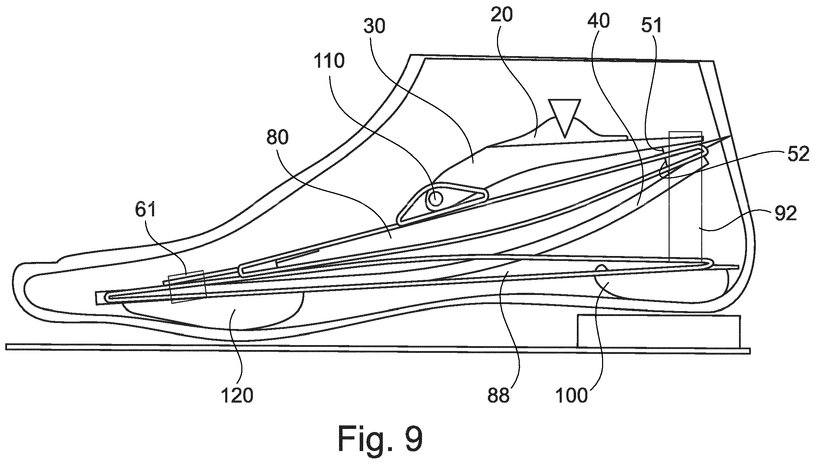

Figur 9 zeigt eine Variante der Figur 8, bei der statt einer Basisfeder ein Bodenführungselement 88 zur Aufnahme und Führung der Hauptfeder 40 angeordnet ist. Das Führungselement 80 und das Bodenführungselement 88 sind im Bereich des Vorderfußpolsters 120 über das Befestigungselement 61 miteinander verbunden, das Führungselement 80 weist an seinem vorderen Ende eine Verlängerungszunge auf, über die eine elastische, federnde Lagerung an dem Bodenführungselement 88 realisiert wird.

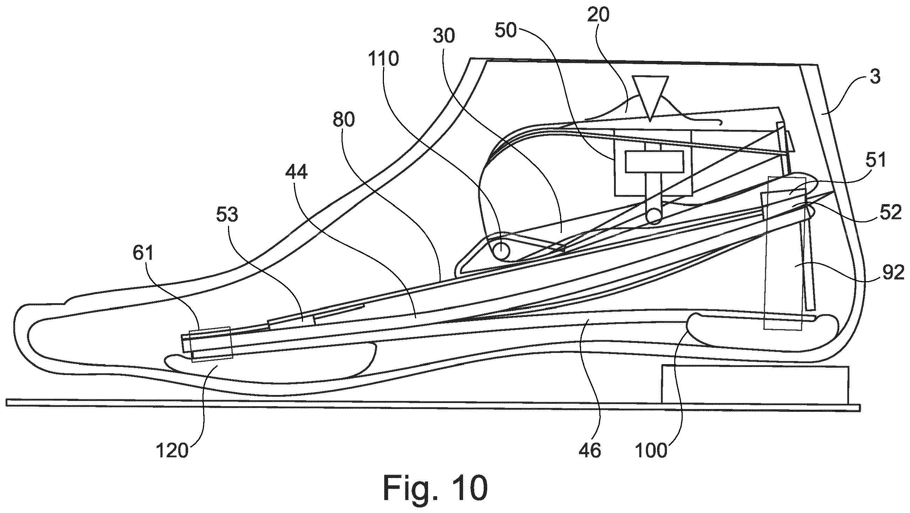

Figur 10 zeigt eine Ausgestaltung einer Variante gemäß Figur 8, bei dem ein Dämpfer 50 zwischen dem Halter 30 und der Befestigungseinrichtung 20 angeordnet ist, sodass nicht nur der Halter 30 um die Schwenkachse 110 relativ zu der Proximalfeder 44 verschwenkbar gelagert ist, sondern auch die Befestigungseinrichtung 20 relativ zu dem Halter 30 um die Schwenkachse 110 verschwenkbar ist. Die Befestigungseinrichtung 20 ist in drei Positionen dargestellt, die durchgezogene Linie stellt die Basiseinstellung dar, die gepunktete Linie eine nach vorn verkippte Position der Befestigungseinrichtung 20 und die gestrichelte Linie eine abgesenkte, nach hinten verkippte Position der Befestigungseinrichtung 20 dar.

Neben einer Ausgestaltung als Dämpfer 50 kann auch ein Aktuator vorgesehen sein, über den eine motorische Verstellung hinsichtlich der Neigung und damit beispielsweise eine Anpassung an unterschiedliche Absatzhöhen erfolgen kann. Ist die Einrichtung als Dämpfer 50 ausgebildet, kann eine konstante Kraft oder ein konstantes Moment dazu führen, dass eine Absenkung oder eine nach Vorwärtsverkippung einstellt. Ein langsames Einsinken oder Anheben ermöglicht eine korrekte Einstellung, beispielsweise durch Schließen entsprechender Stellventile und Verriegeln des als Hydraulikdämpfer ausgebildeten Dämpfers 50 in der gewünschten Position.

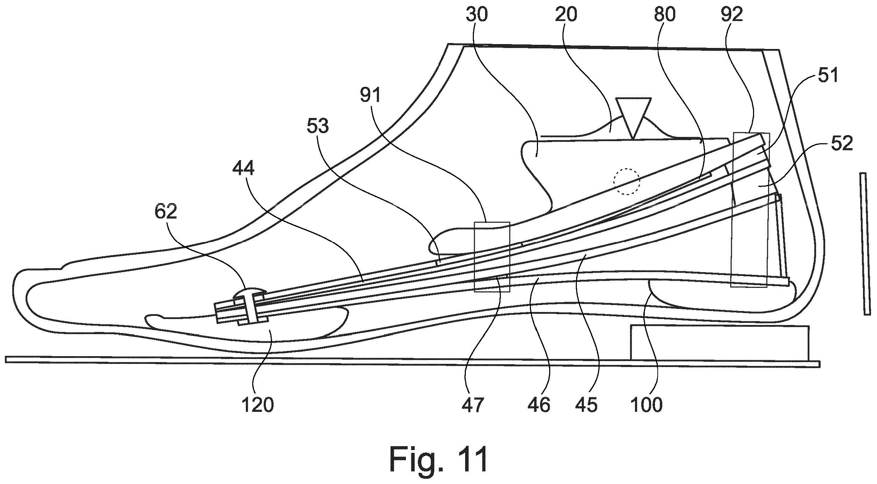

Die Figur 11 zeigt eine Variante der Figur 6 mit einem Halter, der eine Formgebung auf der Unterseite aufweist, die angenähert parallel zu der Formgebung der Oberseite der Proximalfeder 44 aufweist. Das Kontaktelement 47 zwischen der Distalfeder 46 und der Medialfeder 45 ist im Bereich des vorderen Begrenzungselementes 91 angeordnet, die Polster 51, 52 im hinteren Bereich des Prothesenfußeinsatzes sind durch das hintere Begrenzungselement 92 gegen ein seitliches Ausweichen gesichert, eine Führung am hinteren Ende der Distalfeder 46 verhindert ein rückwärtiges Ausweichen der Polster 51, 52. Das Führungselement 80 erstreckt sich von dem hinteren Ende im posterioren Bereich 42 bis zum vorderen Ende der Federn 44, 45, 46 und wird durch die Schraube 62 daran festgelegt. Sowohl die Federn 44, 45, 46 als auch das Führungselement 80 werden durch die Schraube 62 klemmend und formschlüssig gehalten, sodass den Federn bei einer Verformung ein Schubmoment überlagert wird, wodurch sich insgesamt die freie Federlänge verkürzt.

Bei der Ausführungsform gemäß Figur 11 ebenso wie bei allen anderen Ausführungsformen ist es möglich, durch eine Fixierung der Begrenzungselemente 91, 92 oder zumindest eines der Begrenzungselemente 91, 92 an dem Halter 30 und der Distalfeder 46 oder dem Fersenpolster 100 eine Stabilisierung gegen eine Pronation und/oder Supination zu erreichen, da dadurch ausgeschlossen wird, dass sich das jeweilige Begrenzungselement 91, 92 relativ zu dem Halter 30 und/oder der Distalfeder 46 verschiebt, lediglich eine Stauchung wird bei einer entsprechenden Axialbelastung zugelassen.

Im Ausführungsbeispiel gemäß Figur 11 sind die Proximalfeder 44 und die Medialfeder 45 im Wesentlichen parallel gekrümmt geformt, wodurch sich der Einfederraum verringert, jedoch ein weicheres Abrollen insgesamt ermöglicht wird. Die Distal-feder 46 ist von der Medialfeder 45 weg gekrümmt, sodass sich ein in posteriorer Richtung vergrößernder Spalt und Freiraum dazwischen ergibt, der ungefähr bis zu dem vorderen Begrenzungselement 91 oder dem Kontaktelement 47 reicht.

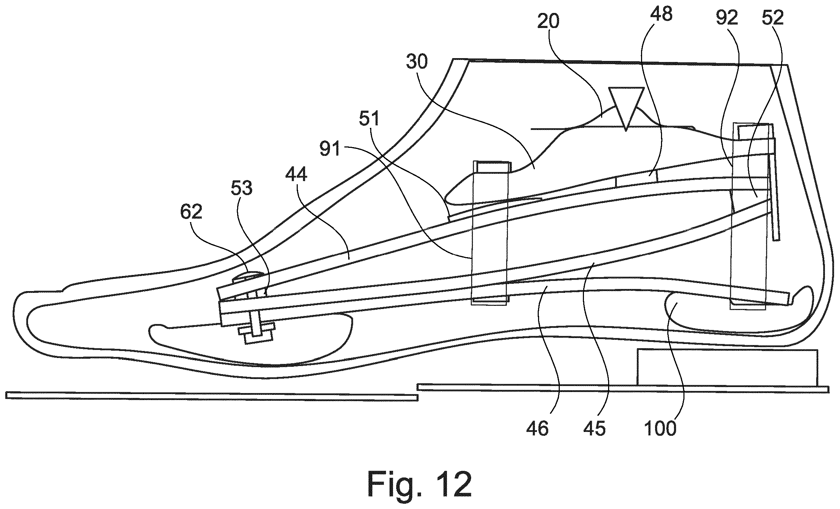

Figur 12 zeigt eine weitere Variante ähnlich der Ausführungsform der Figur 6, jedoch ohne Führungselement und mit einem Polster 51 im anterioren Bereich des Halters 30. Im Bereich unterhalb der Befestigungseinrichtung 20 ist ein Kontaktelement 48 zwischen der Unterseite des Halters 30 und der Oberseite der Proximalfeder 44 angeordnet, die Federn 44, 45, 46 sind über eine Schraube 42 aneinander festgelegt und mit dem Vorderfußpolster 120 gekoppelt. Durch die Positionierung des Kontaktelementes 48 wird die Position des Druckpunktes oder des Krafteinleitungspunktes definiert. Solange eine Fersenlast vorliegt und der Vorfuß nicht aufsetzt, bestimmt der Abrollpunkt der Ferse den Krafteinleitungspunkt. Das hintere Begrenzungselement 92 ist entspannt, die Federn 44, 45, 46 im Fersenbereich sind komprimiert und aufeinander zu verlagert. Das vordere Begrenzungselement 91 ist gespannt und verhindert eine Verlagerung des Halters 30 von der Proximalfeder 44. Sobald der Vorfuß aufsetzt, wird das vordere Begrenzungselement 91, beispielsweise ein Gurt, entspannt, die Distalfeder 46 und das hintere Ende des Halters 30 entfernen sich voneinander und spannen das hintere Begrenzungselement 92 bis zu einer eingestellten Maximalentfernung. Je weiter der Kontaktpunkt zwischen dem Halter 30 und der Proximalfeder 44 nach vorne, also in anteriorer Richtung positioniert ist, desto weicher muss die axiale Nachgiebigkeit eingestellt sein.

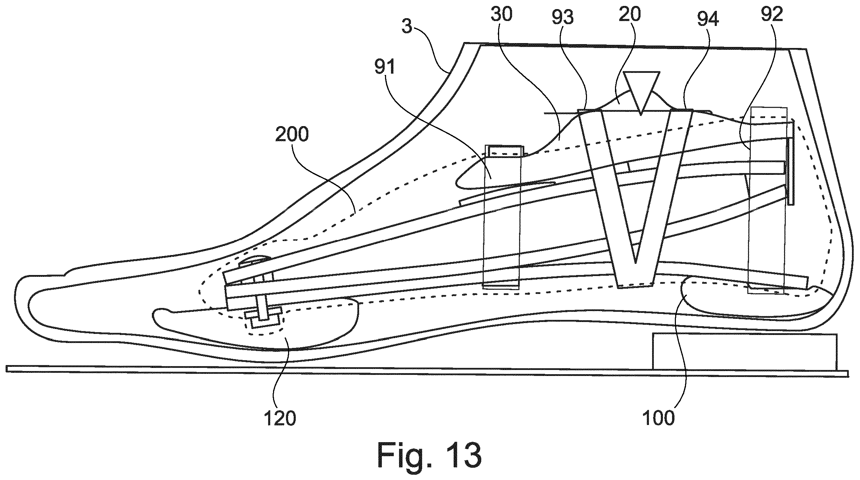

Eine weitere Variante der Erfindung ist in der Figur 13 gezeigt, deren Federaufbau im Wesentlichen dem der Figur 12 entspricht. Zusätzlich zu den Begrenzungselementen 91, 92 ist eine funktionale Umhüllung 200 angeordnet, die um die Federkomponenten und den Halter herum angeordnet ist. Die funktionale Umhüllung 200 ist sockenartig ausgebildet und wird zusätzlich zu der Fußkosmetik 3 verwendet. In dem dargestellten Ausführungsbeispiel umgibt die funktionale Umhüllung 200 die Fersenpolster 100 und Vorderfußpolster 120 nicht, es kann diese aber auch einschließen. Die funktionale Umhüllung 200 kann aus einem undehnbaren oder hochfesten Material mit angepasster Elastizität ausgebildet sein und wird insbesondere dazu verwendet, um Reibung und dadurch Geräusche innerhalb des Prothesenfußes zu verringern. Zusätzliche Gurte oder Spannelemente 93, 94 können zusammen mit der funktionalen Umhüllung 200 verwendet werden, um die elastischen Eigenschaften und die Abroll- sowie Energietransfereigenschaften des Prothesenfußeinsatzes 10 einzustellen, die Spannelemente 93, 94 können auch in der funktionalen Umhüllung 200 integriert sein.

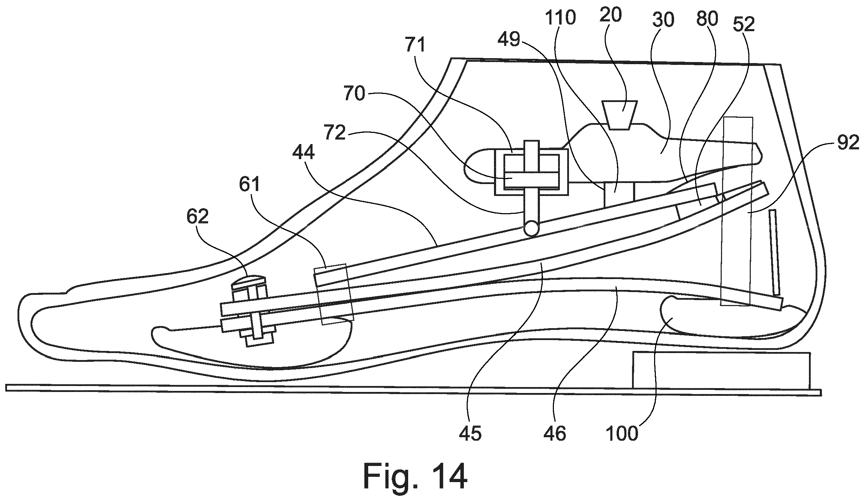

Figur 14 zeigt eine Variante mit einem Halter 30, an dessen vorderen Ende eine Zylinderkammer 71 zur Aufnahme eines beweglichen Kolbens 70 ausgebildet oder angeordnet ist. Der Kolben 70 stützt sich über eine Kolbenstange 72 auf der Proximalfeder 44 ab. Zusätzlich ist der Halter 30 an seinem posterioren Ende über das Führungselement 80 mit dem vorderen Ende der Proximalfeder 44 verbunden. Das Führungselement 80 ist im vorderen Bereich über das Befestigungselement 61 mit der Feder 44 gekoppelt. Die Medialfeder 45 und die Distalfeder 46 sind separat aneinander über die Schraube 62 miteinander verbunden.

Der Halter 30 ist über das Kontaktelement 49 schwenkbar um die Schwenkachse 110 an der Proximalfeder 44 gelagert. Bei einer Fersenbelastung, beispielsweise bei einem Heel Strike, bis zu einer frühen Standphase, bei der der Prothesenfußeinsatz flach auf dem Boden aufliegt, tritt der Kolben 70 innerhalb der Zylinderkammer 71 nicht in Erscheinung, sodass keine Axialkraft über die Kolbenstange 72 auf die Proximalfeder 44 ausgeübt wird. Sobald die Abrollbewegung fortschreitet und eine zunehmende Vorderfußbelastung ausgeführt wird, tritt der Kolben 70 mit der oberen Zylinderkammerbegrenzung in Kontakt und eine Druckkraft wird über die Kolbenstange 72 auf die Federn ausgeübt. Dadurch wird ab einem vorbestimmbaren Zeitpunkt eine Axialkraft in die Hauptfeder 40 eingeleitet und eine weitere Verlagerung in Vorwärtsrichtung oder anteriore Verkippung verhindert oder erschwert, sodass ab der mittleren Standphase eine erhöhte Stabilität für den Nutzer bereitsteht. Ein Abrollen und ein leichtes Verkippen um eine Nulllage herum werden durch das Spiel, das der Kolben 70 innerhalb der Kammer 71 aufweist, ermöglicht. Eine Rückstellkraft kann durch das Führungselement 80 bereitgestellt werden.

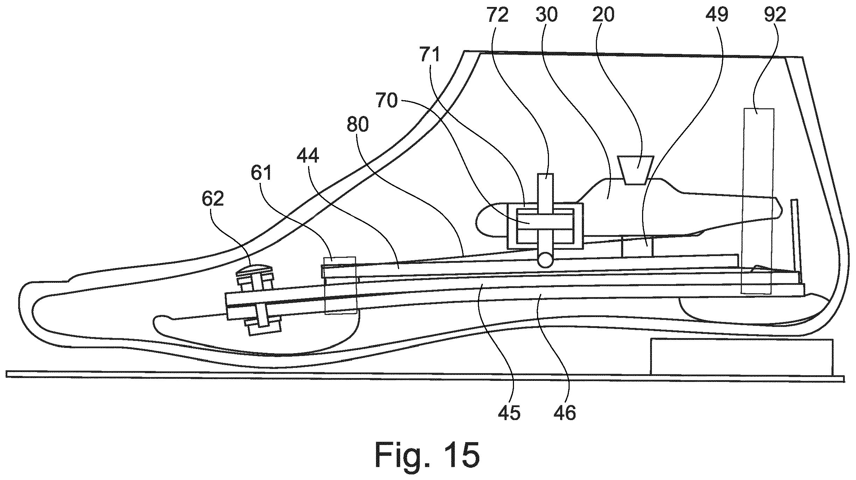

Figur 15 zeigt die Position, bei der bei einer hohen Axialbelastung der Kolben 70 an der Oberseite der Kammer 71 anstößt und eine Druckkraft auf die Federn ausübt. Das hintere Begrenzungselement 92 ist entspannt, das hintere Ende des Führungselementes 80 ist von der Oberseite der Proximalfeder 44 abgehoben.

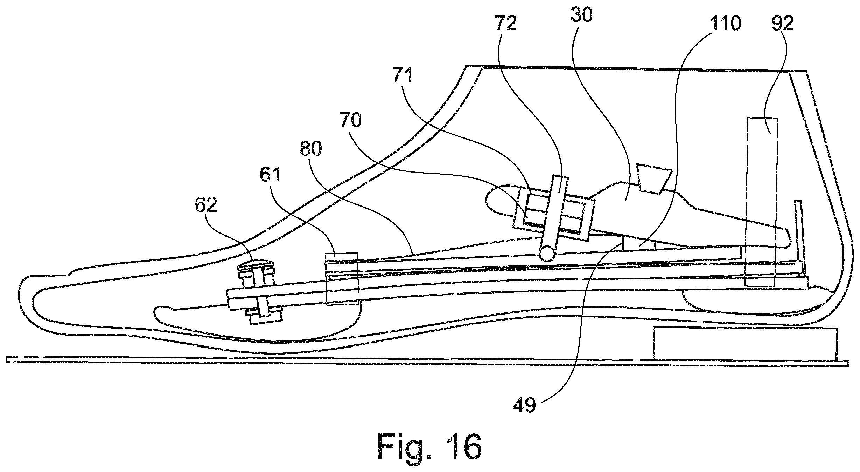

Figur 16 zeigt den Prothesenfußeinsatz gemäß der Figuren 14 und 15 in einem Zustand, in dem der Halter 30 entgegen der Gehrichtung verkippt ist, der Kolben 70 tritt mit der Unterseite der Zylinderkammer 71 in Kontakt und verhindert eine weitere Verschwenkung entgegen der Richtung im Uhrzeigersinn und damit eine weitere Verlagerung des Halters 30 oder des vorderen Endes des Halters 30 von der Proximalfeder 44 weg.

Alle Ausführungsformen der Erfindung ermöglichen es, den Prothesenfußeinsatz 10 vergleichsweise flach auszubilden, sodass es grundsätzlich möglich ist, den Prothesenfußeinsatz 10 zusammen mit einem zusätzlichen Prothesenfußgelenk einzusetzen oder an Patienten mit einem langen Unterschenkelstumpf, beispielsweise Amputationsstumpf anzupassen. Eine geringe Anzahl an Bauteilen, die einfach zu fertigen sind und keine komplexen Formgebungsverfahren benötigen, erleichtern einerseits die Fertigung und andererseits ein robustes und zuverlässiges Design, das zudem einfach von einem Orthopädietechniker an die unterschiedlichen Bedürfnisse und Nutzungsvarianten der jeweiligen Nutzer angepasst werden kann. Die mechanische Auslegung der Prothesenfußeinsätze 10 mit wenig bewegten Teilen benötigt keinen oder nur einen geringen Wartungsaufwand, sodass trotz einer individuellen Anpassbarkeit und gegebenenfalls Veränderbarkeit während der Dauer der Benutzung nur ein geringer Serviceaufwand betrieben werden muss.

Bei den meisten Ausführungsformen der Erfindung sind drei Blattfedern angeordnet, die in zwei Funktionspaare aufgeteilt werden können. Die Proximal- und Medialfeder arbeiten in der Regel als Vorfußfeder, während die Medialfeder und Distalfeder vorwiegend als Fersenfeder arbeiten. Die Distalfeder wird am Ende der Vorfußbewegung noch zugeschaltet. Die Ferse ebenso wie der Vorfuß sind durch die Begrenzungselemente im entlasteten Zustand vorgespannt, ebenso bei einer normalen Standbelastung. Der Halter ist über ein Führungselement an der Federanordnung befestigt, in der Regel an der Proximalfeder. Über zumindest ein Kontaktelement, das zwischen dem Halter und der Proximalfeder angeordnet ist, werden der Krafteinleitungspunkt und damit der Übergang von Fersenlast zur Standphase und Vorfußlast eingestellt. Ein zweiter Kontaktpunkt im Bereich des physiostabilen Punktes anterior der Befestigungseinrichtung hat die Funktion, den Krafteinleitungspunkt bei Vorfußbelastung zu definieren. Durch die Verlagerung der Kontaktpunkte oder der Kontaktelemente zwischen den jeweiligen Federn wird die Federsteifigkeit des Gesamtsystems verändert, ohne dass sich die Zuordnung der einzelnen Federn zueinander und damit der Gesamtaufbau des Prothesenfußeinsatzes verändern. Bei einer normalen Belastung des Prothesenfußes während des Stehens sind die Axialkräfte gleichmäßig auf beide Füße eines Patienten verteilt. In der Regel ist dann die Federvorspannung so groß, dass die beiden Begrenzungselemente oder das posteriore Begrenzungselement noch nicht entspannt sind oder ist. Mit anderen Worten ist die Vorspannung über die Begrenzungselemente oder Vorspannelemente so gewählt, dass während des Stehens eine ausreichende Stabilität über den Prothesenfußeinsatz bereitgestellt wird, um dem Nutzer ein ausreichendes Sicherheitsgefühl zu geben. Bei einer Vorfußbelastung, also bei einem leichten Nachvornebeugen, wird aufgrund der Verlagerbarkeit des Halters um eine Schwenkachse ein sanfter Abrollvorgang eingeleitet, ebenso wird die Energie in den Federn gespeichert und ein Energieübergang von der Fersenfeder zur Vorfußlast ermöglicht.

Darüber hinaus ist es möglich, durch die Anpassung der Federn eine Absenkung in der mittleren Standphase bereitzustellen, wodurch ein gleichmäßiges Einsinken und eine an die natürliche Gangbewegung angepasste Bewegung erreicht werden kann. Die während der mittleren Standphase in den Federn gespeicherte Energie wird bei der weiteren Gangprogression abgegeben und erleichtert die Vorwärtsbewegung.

Insbesondere bei der Landephase des Fußes nach der Schwungphase, also beim Heel Strike, bieten die Prothesenfußeinsätze viel Federweg in der Ferse. Darüber hinaus kann die Federvorspannung über das Begrenzungselement im Fersenbereich vorgespannt werden. Bei einer Vorfußbelastung am Ende der Standphase wirken alle drei Federn oder alle Federn gemeinsam. Aufgrund des Energietransportes beim Abrollen von dem Fersenauftritt in den Vorderfuß am Ende der Standphase wird der Nutzer in Gehrichtung bewegt, da beim Überrollen aufgrund des Einsinkens keine Vertikalbewegung nach oben erfolgt. Daher sind technisch kurze Prothesenfußeinsätze möglich, ohne dass der Nutzer das Gefühl hat, dass er am Ende der Standphase in ein Loch fällt, da sich bei mechanisch kurzen Füßen ein unnatürliches frühes Abrollen und ein Absenken des Körperschwerpunktes ergibt. Mit den Prothesenfußeinsätzen wird eine Absenkung in der mittleren Standphase durch die gleichmäßige Federvorspannung bewirkt, die am Ende der Standphase in ein Anheben resultiert, wenn sich die Federn im Vorderfuß entspannen.

Neben der Position der Kontaktelemente sind auch die Form und die Abmessung der Kontaktelemente entscheidend für das Gangverhalten und den Energietransport. Je schmaler die Kontaktelemente sind und damit die Krafteinleitungsbereiche, desto präziser und genauer ist das Verhalten, je breiter die Kontaktelemente oder Krafteinleitungsbereiche sind, desto weicher ist das Ganggefühl.

Bevorzugt sind die Federn senkrecht oder nahezu senkrecht zu der Belastung orientiert, bei Blattfedern ist somit die Längserstreckung im Wesentlichen senkrecht zu der Belastungsrichtung, wodurch die Materialeigenschaften der Blattfedern optimal ausgenutzt werden. Die Vorspannung der Federn wird vorteilhafterweise so gewählt, dass beim normalen Stehen keine oder nur eine minimale Verformung auftritt, sodass der Krafteinleitungspunkt stabil bleibt und ein ruhiges Stehen ohne übermäßige Steifheit möglich ist.

### Bezugszeichenliste

- 2 -: Proximalkomponente
- 3 -: Fußkosmetik
- 10 -: Prothesenfußeinsatz
- 20 -: Befestigungseinrichtung
- 30 -: Halter
- 40 -: Hauptfeder
- 41 -: anteriorer Bereich
- 42 -: posteriorer Bereich
- 43 -: Basisfeder
- 44 -: Proximalfeder
- 45 -: Medialfeder
- 46 -: Distalfeder
- 47 -: Kontaktelement
- 48 -: Kontaktelement
- 49 -: Kontaktelement
- 50 -: Dämpfer
- 51 -: Dämpfer
- 52 -: Dämpfer
- 53 -: Polster
- 54 -: Polster
- 55 -: Polster
- 60 -: Schiebelager
- 61 -: Befestigungselement
- 62 -: Schraube
- 70 -: Kolben
- 71 -: Zylinder
- 72 -: Kolbenstange
- 80 -: Führungselement
- 88 -: Bodenführungselement
- 91 -: Begrenzungselement
- 92 -: Begrenzungselement
- 100 -: Fersenpolster
- 110 -: Schwenkachse
- 111 -: Schwenkachse
- 120 -: Vorderfußpolster
- 200 -: Umhüllung
- 400 -: Freiraum
- 401 -: Freiraum
- 410 -: Krafteinleitungsbereich
- 420 -: Krafteinleitungsbereich
- 910 -: Aufnahme
- 920 -: Aufnahme
- 930 -: Aufnahme
- 940 -: Aufnahme

## Patentansprüche

1. Prothesenfußeinsatz (10) mit
a. einer proximalen Befestigungseinrichtung (20) zum Festlegen des Prothesenfußeinsatzes (10) an einer Proximalkomponente (2) oder einem Patienten,
b. einem distal zu der Befestigungseinrichtung (20) angeordneten und mit der Befestigungseinrichtung (20) verbundenen Halter (30), und
c. einer Hauptfeder (40), die sich in einen Vorderfußbereich (11) erstreckt und mit dem Halter (30) gekoppelt ist,
d. der Halter (30) in der Sagittalebene kippbar an der Hauptfeder (40) gelagert ist, wobei ein posteriores Begrenzungselement (92) zwischen der Hauptfeder (40) und dem Halter (30) angeordnet ist, das eine Verlagerung des Halters (30) von der Hauptfeder (40) weg begrenzt, **dadurch gekennzeichnet, dass** die Hauptfeder (40) als zusammengesetzte Feder mit zumindest einer Proximalfeder (44) und zumindest einer Distalfeder (45, 46) ausgebildet ist und die Proximalfeder (44) und die Distalfeder (45, 46) oder die Distalfedern (45, 46) bikonvex geformt zueinander ausgerichtet sind .

2. Prothesenfußeinsatz nach Anspruch 1, **dadurch gekennzeichnet, dass** die Proximalfeder (44) und die Distalfeder (45, 46) unter Ausbildung eines Freiraumes (400, 401) beabstandet zueinander aneinander festgelegt sind.

3. Prothesenfußeinsatz nach einem der voranstehenden Ansprüche, **dadurch gekennzeichnet, dass** ein Führungselement (80) an der Hauptfeder (40) in einem anterioren Bereich (41) oder posterioren Bereich (42) befestigt ist und sich in eine jeweils entgegengesetzte Richtung erstreckt und der Halter (30) über das Führungselement (80) verkippbar gelagert ist.

4. Prothesenfußeinsatz nach Anspruch 3, **dadurch gekennzeichnet, dass** ein anteriores Begrenzungselement (91) zwischen dem Führungselement (80) und dem Halter (30) oder der Hauptfeder (40) angeordnet ist, das bei einer Fersenbelastung eine Verlagerung eines anterioren Endes des Halters (30) oder eine Verlagerung der Hauptfeder (40) von dem Führungselement (80) weg begrenzt.

5. Prothesenfußeinsatz nach Anspruch 3 oder 4, **dadurch gekennzeichnet, dass** die Hauptfeder (40) und das Führungselement (80) als Blattfedern ausgebildet sind.

6. Prothesenfußeinsatz nach einem der voranstehenden Ansprüche, **dadurch gekennzeichnet, dass** das Begrenzungselement (91, 92) zugstarr und flexibel ausgebildet ist.

7. Prothesenfußeinsatz nach einem der voranstehenden Ansprüche, **dadurch gekennzeichnet, dass** an der Hauptfeder (40) ein Vorderfußpolster (120) und/oder ein Fersenpolster (100) befestigt ist.

8. Prothesenfußeinsatz nach einem der voranstehenden Ansprüche, **dadurch gekennzeichnet, dass** der Halter (30) über eine Federzunge, ein Scharnier oder zumindest ein Abstandselement auf der Hauptfeder (40) gelagert ist.

9. Prothesenfußeinsatz nach einem der voranstehenden Ansprüche, **dadurch gekennzeichnet, dass** der Halter (30) Kräfte über einen anterioren Krafteinleitungsbereich (410) anterior der Befestigungseinrichtung (20) und einen posterioren Krafteinleitungsbereich (420) posterior der Befestigungseinrichtung (20) in die Hauptfeder (40) einleitet.

10. Prothesenfußeinsatz nach Anspruch 9, **dadurch gekennzeichnet, dass** zumindest einer der Krafteinleitungsbereiche (410, 420) verlagerbar oder auswechselbar an dem Halter (30) oder der Hauptfeder (40) gelagert ist.

11. Prothesenfußeinsatz nach einem der voranstehenden Ansprüche, **dadurch gekennzeichnet, dass** zwischen dem Halter (30) und der Hauptfeder (40) zumindest eine Dämpfereinrichtung (51, 52) angeordnet ist.

12. Prothesenfußeinsatz nach einem der voranstehenden Ansprüche, **dadurch gekennzeichnet, dass** der Halter (30) mit einem einstellbaren proximal-distal-Abstand an der Hauptfeder (40) gelagert ist.

13. Prothesenfußeinsatz nach Anspruch 3 oder 4, **dadurch gekennzeichnet, dass** im unbelasteten Zustand die Hauptfeder (40) gegenüber dem Führungselement (80) durch das Begrenzungselement (91, 92) elastisch vorgespannt ist.

14. Prothesenfußeinsatz nach einem der voranstehenden Ansprüche, **dadurch gekennzeichnet, dass** die Befestigungseinrichtung (20) verschieblich und/oder gelenkig an dem Halter (30) gelagert ist.

15. Prothesenfußeinsatz nach Anspruch 13, **dadurch gekennzeichnet, dass** zwischen der Befestigungseinrichtung (20) und dem Halter (30) ein Dämpfer (50) angeordnet ist.

16. Prothesenfußeinsatz nach einem der voranstehenden Ansprüche, **dadurch gekennzeichnet, dass** zwischen der Proximalfeder (44) und der Distalfeder (45) und/oder zwischen den Distalfedern (45, 46) ein auswechselbares und/oder verschieblich gelagertes Kontaktelement (47, 48, 49) angeordnet ist.

17. Prothesenfußeinsatz nach einem der voranstehenden Ansprüche, **dadurch gekennzeichnet, dass** der Halter (30) in der Sagittalebene auf einer ortsveränderlichen Fläche kippbar an der Hauptfeder (40) gelagert ist.

## Claims

1. A prosthetic foot insert (10) with
a. a proximal fastening device (20) for securing the prosthetic foot insert (10) to a proximal component (2) or to a patient,
b. a holder (30) which is arranged distally with respect to the fastening device (20) and is connected to the fastening device (20), and
c. a main spring (40) which extends into a forefoot region (11) and is coupled to the holder (30), **characterized in that**
the holder (30) is mounted on the main spring (40) so as to be tiltable in the sagittal plane, wherein a posterior limiting element (92) is arranged between the main spring (40) and the holder (30) and limits a displacement of the holder (30) away from the main spring (40) **characterized in that** the main spring (40) is designed as a combined spring having at least one proximal spring (44) and at least one distal spring (45, 46)and that the proximal spring (44) and the distal spring (45, 46) or the distal springs (45, 46) are oriented relative to each other in a biconvex shape.

2. The prosthetic foot insert as claimed in claim 1, **characterized in that** the proximal spring (44) and the distal spring (45, 46) are secured to each other at points spaced apart from each other so as to form a free space (400, 401).

3. The prosthetic foot insert as claimed in one of the preceding claims, **characterized in that** a guide element (80) is fastened to the main spring (40) in an anterior region (41) or posterior region (42) and extends in a respectively opposite direction, and the holder (30) is mounted so as to be tiltable via the guide element (80).

4. The prosthetic foot insert as claimed in claim 3, **characterized in that** an anterior limiting element (91) is arranged between the guide element (80) and the holder (30) or the main spring (40), which limiting element (91), in the case of a heel load, limits a displacement of an anterior end of the holder (30) or a displacement of the main spring (40) away from the guide element (80).

5. The prosthetic foot insert as claimed in claim 3 or 4, **characterized in that** the main spring (40) and the guide element (80) are designed as leaf springs.

6. The prosthetic foot insert as claimed in one of the preceding claims, **characterized in that** the limiting element (91, 92) is tensionally rigid and flexible.

7. The prosthetic foot insert as claimed in one of the preceding claims, **characterized in that** a forefoot pad (120) and/or a heel pad (100) is fastened to the main spring (40).

8. The prosthetic foot insert as claimed in one of the preceding claims, **characterized in that** the holder (30) is mounted on the main spring (40) via a spring tongue, a hinge or at least one spacer element.

9. The prosthetic foot insert as claimed in one of the preceding claims, **characterized in that** the holder (30) introduces forces into the main spring (40) via an anterior force introduction region (410) anterior to the fastening device (20) and a posterior force introduction region (420) posterior to the fastening device (20).

10. The prosthetic foot insert as claimed in claim 9, **characterized in that** at least one of the force introduction regions (410, 420) is mounted displaceably or exchangeably on the holder (30) or the main spring (40).

11. The prosthetic foot insert as claimed in one of the preceding claims, **characterized in that** at least one damper device (51, 52) is arranged between the holder (30) and the main spring (40).

12. The prosthetic foot insert as claimed in one of the preceding claims, **characterized in that** the holder (30) is mounted on the main spring (40) with an adjustable proximal-distal distance.

13. The prosthetic foot insert as claimed in claim 3 or 4, **characterized in that**, in the unloaded state, the main spring (40) is elastically pretensioned with respect to the guide element (80) by the limiting element (91, 92).

14. The prosthetic foot insert as claimed in one of the preceding claims, **characterized in that** the fastening device (20) is mounted on the holder (30) in a displaceable and/or articulated manner.

15. The prosthetic foot insert as claimed in claim 13, **characterized in that** a damper (50) is arranged between the fastening device (20) and the holder (30).

16. The prosthetic foot insert as claimed in one of the preceding claims, **characterized in that** an exchangeable and/or displaceably mounted contact element (47, 48, 49) is arranged between the proximal spring (44) and the distal spring (45) and/or between the distal springs (45, 46).

17. The prosthetic foot insert as claimed in one of the preceding claims, **characterized in that** the holder (30) is mounted on the main spring (40) so as to be tiltable in the sagittal plane on a movable surface.

## Revendications

1. Insert de prothèse de pied (10) comprenant
a. un dispositif de fixation proximal (20) destiné à fixer l'insert de prothèse de pied (10) à un composant proximal (2) ou à un patient,
b. un support (30) disposé en position distale par rapport au dispositif de fixation (20) et relié au dispositif de fixation (20), et
c. un ressort principal (40) qui s'étend dans une zone de l'avant-pied (11) et qui est couplé au support (30),
d. le support (30) étant monté sur le ressort principal (40) de manière à pouvoir basculer dans le plan sagittal, un élément de limitation postérieur (92) étant disposé entre le ressort principal (40) et le support (30), lequel limite un déplacement du support (30) s'éloignant du ressort principal (40),
**caractérisé en ce que**
le ressort principal (40) est conçu comme un ressort composite comprenant au moins un ressort proximal (44) et au moins un ressort distal (45, 46), et le ressort proximal (44) et le ressort distal (45, 46) ou les ressorts distaux (45, 46) sont alignés l'un par rapport à l'autre en étant de forme biconvexe.

2. Insert de prothèse de pied selon la revendication 1,
**caractérisé en ce que** le ressort proximal (44) et le ressort distal (45, 46) sont fixés l'un à l'autre en étant espacés l'un de l'autre de manière à former un espace libre (400, 401).

3. Insert de prothèse de pied selon l'une des revendications précédentes, **caractérisé en ce qu'**un élément de guidage (80) est fixé au ressort principal (40) dans une zone antérieure (41) ou dans une zone postérieure (42) et s'étend dans une direction opposée respective, et le support (30) est monté de manière à pouvoir basculer par l'intermédiaire de l'élément de guidage (80).

4. Insert de prothèse de pied selon la revendication 3,
**caractérisé en ce qu'**un élément de limitation antérieur (91) est disposé entre l'élément de guidage (80) et le support (30) ou le ressort principal (40), lequel limite, lors d'une mise en charge du talon, un déplacement d'une extrémité antérieure du support (30) ou un déplacement du ressort principal (40) s'éloignant de l'élément de guidage (80).

5. Insert de prothèse de pied selon la revendication 3 ou 4,
**caractérisé en ce que** le ressort principal (40) et l'élément de guidage (80) sont réalisés sous forme de ressorts à lames.

6. Insert de prothèse de pied selon l'une des revendications précédentes, **caractérisé en ce que** l'élément de limitation (91, 92) est réalisé de façon rigide en traction et flexible.

7. Insert de prothèse de pied selon l'une des revendications précédentes, **caractérisé en ce qu'**un coussin d'avant-pied (120) et/ou un coussin de talon (100) est fixé au ressort principal (40).

8. Insert de prothèse de pied selon l'une des revendications précédentes, **caractérisé en ce que** le support (30) est monté sur le ressort principal (40) par l'intermédiaire d'une languette élastique, d'une charnière ou d'au moins un élément d'écartement.

9. Insert de prothèse de pied selon l'une des revendications précédentes, **caractérisé en ce que** le support (30) applique des forces au ressort principal (40) par l'intermédiaire d'une zone d'application de force antérieure (410) située du côté antérieur du dispositif de fixation (20) et par l'intermédiaire d'une zone d'application de force postérieure (420) située du côté postérieur du dispositif de fixation (20).

10. Insert de prothèse de pied selon la revendication 9,
**caractérisé en ce que** l'une au moins des zones d'application de force (410, 420) est montée de manière déplaçable ou interchangeable sur le support (30) ou sur le ressort principal (40).

11. Insert de prothèse de pied selon l'une des revendications précédentes, **caractérisé en ce qu'**au moins un dispositif d'amortissement (51, 52) est disposé entre le support (30) et le ressort principal (40).

12. Insert de prothèse de pied selon l'une des revendications précédentes, **caractérisé en ce que** le support (30) est monté sur le ressort principal (40) avec un écartement proximal-distal réglable.

13. Insert de prothèse de pied selon la revendication 3 ou 4,
**caractérisé en ce que**, à l'état non chargé, le ressort principal (40) est précontraint élastiquement par rapport à l'élément de guidage (80) au moyen de l'élément de limitation (91, 92).

14. Insert de prothèse de pied selon l'une des revendications précédentes, **caractérisé en ce que** le dispositif de fixation (20) est monté de manière coulissante et/ou articulée sur le support (30).

15. Insert de prothèse de pied selon la revendication 13,
**caractérisé en ce qu'**un amortisseur (50) est disposé entre le dispositif de fixation (20) et le support (30).

16. Insert de prothèse de pied selon l'une des revendications précédentes, **caractérisé en ce qu'**un élément de contact (47, 48, 49) interchangeable et/ou monté de manière coulissante est disposé entre le ressort proximal (44) et le ressort distal (45) et/ou entre les ressorts distaux (45, 46).

17. Insert de prothèse de pied selon l'une des revendications précédentes, **caractérisé en ce que** le support (30) est monté sur le ressort principal (40) de manière à pouvoir basculer dans le plan sagittal sur une surface mobile.
